# EUROPEAN PATENT SPECIFICATION

(11) **EP 2 421 404 B1**
(45) Date of publication and mention of the grant of the patent: **07.05.2014**
(21) Application number: 10767654.6
(22) Date of filing: 20.04.2010
(51) Int. Cl.: B05B 7/08, A61M 35/00, B05B 13/04, B05B 15/12

(54) **AUTOMATED SKIN SPRAY AND DRY SYSTEM**
AUTOMATISCHES HAUTBESPRÜH- UND TROCKNUNGSSYSTEM
PULVÉRISATEUR DE PEAU AUTOMATIQUE ET SYSTÈME SEC

(30) Priority: 21.04.2009 US 171160 P
(43) Date of publication of application: 29.02.2012
(73) Proprietor: MT Industries, Inc., Macedonia, OH 44056 (US)
(72) Inventor: COOPER, Steven, C., Athens GA 30605 (US); CROFT, Ricky, C., Coppell TX 75019 (US)
(74) Representative: Konkonen, Tomi-Matti Juhani
(86) International application number: PCT/US2010/031779
(87) International publication number: WO 2010/123922

(56) References cited:
- GB-A- 2 139 885
- GB-A- 2 432 785
- US-A- 4 505 229
- US-A1- 2005 281 957
- US-A1- 2007 169 261
- US-A1- 2007 197 982
- US-B1- 6 554 208
- US-B1- 6 554 208

## Description

### PRIORITY CLAIM

This application claims the benefit of United States Application for Patent No. 12/763,535 filed April 20, 2010, which claims priority from United States Provisional Application for Patent.No. 61/171,160 filed April 21, 2009.

### BACKGROUND

### Technical Field

The present invention relates to a system that uniformly delivers human body coating compositions (such as skin treatment solutions) and provides for drying of the delivered coating compositions.

### History of Related Art

Spray devices for the application of liquids onto human skin and hair are well known. See for example GB-A-2 432 785, GB-A-2 139 885 or US-B-6 554 208. Spray are used for many types of medicines, skin treatments, hair treatments, deodorants, lotions, and cosmetic agents. Recently, automated spray systems have been introduced and are used primarily by tanning salons for the application of sunless tanning liquids and skin care products, such as moisturizers and exfoliants. An advantage of an automated system is that such a system allows for privacy and reduced operating costs.

At the present time the widest usage of automated skin treatment spray systems is for sunless tanning in tanning salons. The spray solution used is generally a water-based mixture of DHA (dihydroxyacetone) and/or erythrulose and various other skin care ingredients such as aloe vera. Often a cosmetic bronzer is added along with various scents and ingredients to enhance tanning performance, such as formulations to balance skin ph.

The automated spray process has been inherently a chilly, uncomfortable experience for the customer. Nozzle expansion cools the air and liquid in the spray cloud significantly. Temperatures of the spray cloud can be over 30 degrees (F) lower than human body temperature. Heating of the spray liquid prior to spraying has a negligible effect on increasing spray cloud temperature due to the rapid cooling produced as spray expands when exiting the nozzle. This phenomenon is magnified when using air-atomizing nozzles; the nozzle type most desirable for producing a well atomized spray for application to human skin.

In an automated spray tanning operation, the user disrobes for the spray treatment which lasts from 30 seconds to 2 minutes. Some sunless machines offer multiple sessions of alternate ingredients so the experience can be significantly longer. Exiting the automated spray booth with wet skin is uncomfortable since tanning salons often keep thermostats low to offset the heat created by their UV tanning beds. Furthermore, cold skin is known to prevent optimum absorption of the skin care ingredients.

After using an automated spray booth, customers often use a towel to dry their skin. The action of toweling off removes a significant quantity of the sprayed ingredients from the skin. The remaining sprayed ingredients may by re-distributed, which can produce a splotchy appearance in the case of sunless tanning or other cosmetic treatment or skin treatment. If the customer opts not to use a towel, and simply dry off in the ambient air or from the cool air supplied by the air-atomizing nozzles themselves, the surface of the skin can become sticky and the customer can become chilled.

Many tanning salons providing the automated spray service also have conventional UV lamp tanning beds. Customers have observed that using the automated sunless tanning spray booths quickly after they use a UV tanning bed can result in a deeper and darker DHA tan. It is important to move from the UV tanning bed to the spray booth as quickly as possible. It is also essential to remove all perspiration resulting from the UV treatment or the tan result can be uneven. The benefits of UV tanning coupled with a sunless tanning spray may be due to opening the pores of the skin and from more thoroughly and more deeply drying out of the top skin layer by the hot UV lamps. However, due to skin health concerns, many customers do not wish to use the UV beds and therefore cannot take advantage of this practice to enhance their sunless tan.

DHA tans the skin by reacting with proteins in the stratum corneum, the top protective skin layer composed of dead skin cells. It is known that only the uppermost dry layers of the stratum corneum will tan effectively with DHA or erythrulose. Very dry skin may pigment the darkest. Skin care specialists suggest using a warm towel on the skin before application of treatments since warm skin absorbs some ingredients better. However, a skin surface that is too hot will perspire, and the presence of perspiration on the skin can reduce the effectiveness of the sprayed ingredients.

There is a need to make the sunless tanning experience with automated spray equipment more comfortable and effective. The present invention provides an automated system that applies spray to the body while incorporating a heated air system that is controlled to provide dry heated air to the skin (for example, simultaneously with spraying or in the form of pre-heat and drying cycles) to enhance the performance of the spray materials and provide improved comfort for the user. It also noted that customer comfort and safety is improved with the present invention by the introduction of clean air (for example, fresh air from outside the enclosure or filtered air) before, during and after the spraying experience.

### SUMMARY

It is an object of the present invention to provide a method and an apparatus for spraying skin treatment spray solution and drying the sprayed skin treatment spray solution. This object can be achieved by the features of the independent claims. Further embodiments are characterized by the dependent claims.

One aspect of the invention relates to a method and apparatus for automating the spray coating process using a spray system to apply a spray coat and a drying system to apply dry heated air. Another aspect of the invention is to provide a heated spray environment to enhance the comfort of the experience before, during and after the spray process. Such an environment could, for example, be provided with an enclosure or booth, although a fully enclosed environment is not required. Another aspect of the present invention is to make multiple passes of the spray cloud with warm dry air applied after each individual spray pass.

These aspects have been found to improve coating uniformity, as well as improve the characteristic feel of the spray deposit on the skin, both during and after the spray session. In the case of DHA applications for sunless tanning, these aspects result in improved tanning color and longevity of the tan.

The controlled application of warm dry air over the skin during and/or between applications of sprays in an automated booth for sunless tanning enhances the efficacy of the tanning compounds resulting in a deeper tan color and a longer lasting tan. In addition, the mixing of heated dry air into the spray cloud reduces the temperature discomfort caused by the inherently cold spray stream and cloud.

In one preferred application process, short multiple passes of the spray cloud are provided, each being followed by application of warmed dry air for a short skin-drying cycle that is effectuated between the individual spray passes. The application of warm dry air during and/or after each spray sequence has been noted to produce a softer characteristic feel of the spray ingredients on the skin and customer complaints of "stickiness" are reduced. Furthermore, an improvement in deposition efficiency and uniformity of the tan result are experienced (especially since the need for any towel drying step after the spray session has been obviated).

In an embodiment, a method comprises: a) spraying a human target with a skin treatment spray solution; b) terminating spraying; c) blowing air on the human target to dry the skin treatment spray solution; d) repeating steps a)-c) a desired number of times; and performing steps a)-d) under the control of an automated control system.

In another embodiment, an apparatus comprises: a spray system for spraying a skin treatment spray solution towards a human target; a forced air system for blowing air towards the human target; an automated control system which is configured to operate to: a) cause the spray system to spray the human target with the skin treatment spray solution; b) terminate operation of the spray system; c) activate the forced air system to blow air on the human target to dry the skin treatment spray solution; and d) repeat operations a)-c) a desired number of times.

The above summary is not intended to represent each embodiment or every aspect of the present invention.

### BRIEF DESCRIPTION OF THE DRAWINGS

A more complete understanding of the method and system of the present invention may be obtained by reference to the following Detailed Description when taken in conjunction with the accompanying Drawings wherein:
FIGURE 1A is a perspective view of a spray booth enclosure;
FIGURE 1B is a top view of the spray booth enclosure;
FIGURE 1C is a front view of the spray system and air outlet used within the spray booth enclosure;
FIGURE 2 is a flow direction for an exemplary operation of the spray system;
FIGURE 3 is a close-up view of the heated forced air system and spray system within the enclosure of FIGURE 1;
FIGURE 4 is a close-up view of an alternative implementation of the heated forced air system and spray system within the enclosure;
FIGURE 5 illustrates a graph of temperature control in relation to fan speed; and
FIGURE 6 illustrates a graph of final temperature stabilization using an exhaust fan.

### DETAILED DESCRIPTION OF THE DRAWINGS

Embodiment(s) of the invention will now be described more fully with reference to the accompanying Drawings. The invention may, however, be embodied in many different forms and should not be construed as limited to the embodiment(s) set forth herein. The invention should only be considered limited by the claims and the equivalents thereof.

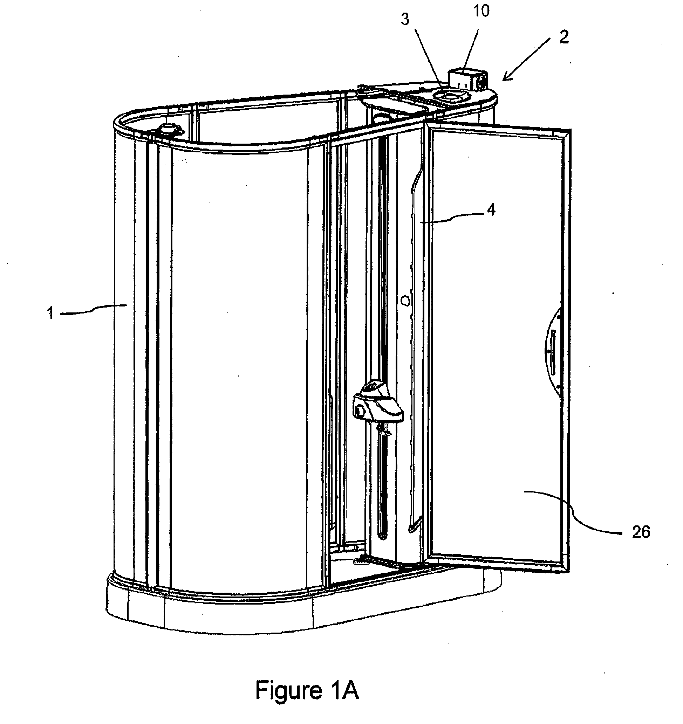

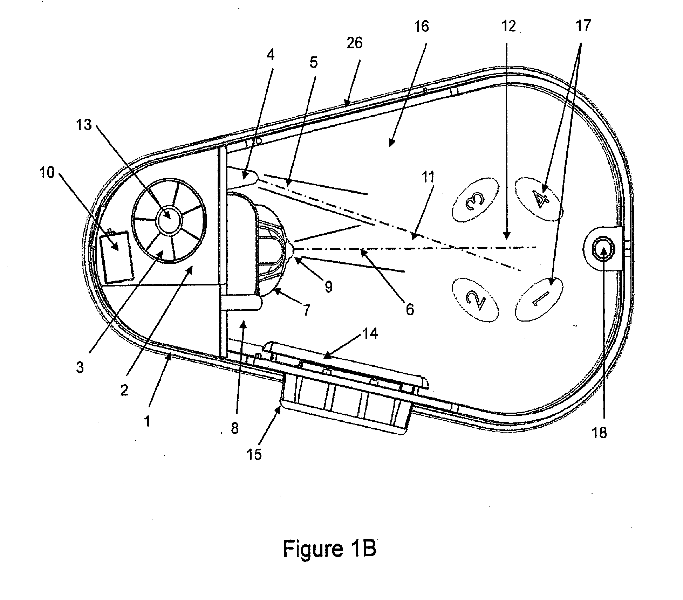

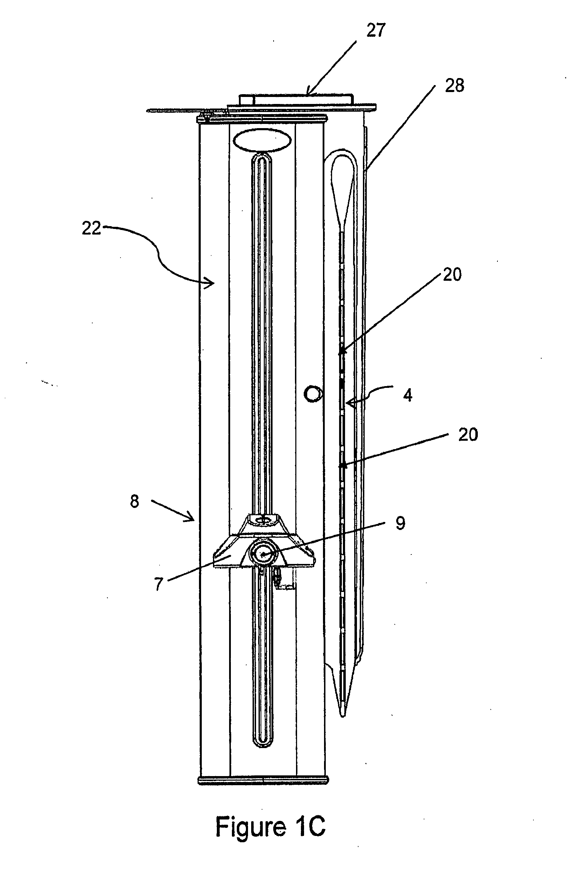

Reference is now made to FIGURES 1A and 1B which show a perspective view and top view, respectively, of a spray booth enclosure. The enclosure includes a set of peripheral walls 1. In FIGURES 1A and 1B, the floor 16 of the enclosure is visible, but the roof of the enclosure has been removed (inclusion of a roof being optional, but preferred). A heated forced air system 2 is provided and enclosed by a wall panel at one end of the enclosure. The system 2 includes a fan 13 receiving air through an air inlet 3. A filter 27 (see, FIGURE 1C) is provided for the air inlet 3 to filter outside air prior to being processed by the heated forced air system 2. A heating element (not explicitly shown) is included within the system 2, and this heating element functions to heat the air received through the air inlet 3. The fan 13 sucks the air in through the inlet and forces the air to pass over the heating element. The heated air is forced by the fan 13 through a vertical slot air outlet 4 provided with an opening in the wall panel to form a warm air stream 5 delivered to the inside of the enclosure. More specifically, the air outlet 4 is configured to direct the warm air stream towards an area 12 where the human target is standing, the air outlet being directed to provide coverage over the length of the human target. A number of standing indicators 17 are provided on the floor of the enclosure to encourage the human target to stand at a desired location within the enclosure. The multiple standing indicators 17 further assist in positioning the human target with a desired orientation.

Reference is now additionally made to FIGURE 1C which shows a front view of the spray system and air outlet used within the spray booth enclosure. Although only one vertical arrangement of an air outlet 4 with multiple slots 20 is shown in FIGURE 1C, it will be understood that multiple vertical air outlets 4, each with slots 20, may be provided. Furthermore, the multiple slots 20 can instead be implemented with a single vertical slot for each outlet 4 to provide a warm air stream 5 having a curtain-like configuration that is directed to provide coverage over the length of the human target standing in the area 12. The air outlet is preferably formed, as shown in FIGURE 1C, by a plurality of individual air openings 20 aligned in a vertical column along the wall panel within the enclosure so as to provide multiple vertically arranged warm air streams 5 directed to provide coverage over the length of the human target standing in the area 12. Additionally, a vane or louver may be provided with the air outlet 4 for controlling the direction of the warm air stream at and along the length of the human target and further controlling where the intersection of the air stream with a spray cloud (as will be described) is made.

The heated forced air system 2 further supports the provision of air having a temperature gradient. For example, the system 2 and air outlet 4 are configured so that air of differing temperature can be delivered by the multiple slots 20. In a preferred implementation, slots 20 which are higher on the air outlet 4 deliver air at a temperature which is lower than the temperature of the air delivered by slots 20 located lower on the air outlet 4. A linear gradient with respect to temperature can be supported. Alternatively, a stepped (or staircase) gradient with respect to temperature can be supported. The advantage of such a temperature gradient heated forced air system 2 is that air that is relatively cooler can be supplied higher and towards the face of the human target, while air that is relatively warmer can be supplied lower and towards the torso and legs of the human target. This temperature gradient enhances customer comfort by supplying cooler and refreshing air to the human target in the face area, thus reducing the perception of stuffiness within the enclosure during operation of the overall system.

At the same end of the enclosure as the heated forced air system 2, a spray system 8 is provided mounted to the wall panel. The spray system 8 includes a nozzle housing 7 with that nozzle housing supporting at least one spray nozzle 9 generating a spray cloud 6 which intersects the warm air stream 5 at or about the region 11 near to the location of the human target. It will thus be noted that the warm air stream and spray cloud are supplied toward the human target from generally the same direction (as shown from the same end of the enclosure). The spray nozzle is moveable so as to direct the spray cloud and delivery of spray material. In one embodiment, the spray nozzle may be actuated for movement to oscillate during the spray operation. For example, the oscillating spray nozzle 9 may be configured for either or both vertical and/or horizontal oscillation. The nozzle 9 may further be configured to support electrostatic charging of the spray (through the use of contact or inductive methods known in the art, and for example as shown in United States Published Patent Application No. 2006/0124780). The spray system 8 may, for example, comprise the spray system with an oscillating nozzle assembly as disclosed in United States Patent No. 7,297,211 and United States Published Patent Application No. 2006/0278661.

Although only one spray nozzle 9 is shown in FIGURE 1, it will be understood that multiple spray nozzles 9 may be provided, for example in substantially vertical alignment within the enclosure, so as to provide multiple spray clouds 6 directed to provide coverage over the length of the human target standing in the area 12. Each of these nozzles may, if desired, horizontally oscillate to provide for enhanced coverage.

It will further be understood that the spray system 8 could be mounted on a vertically traveling gantry 22, with the nozzle vertically traversing during spray operation (perhaps while simultaneously horizontally oscillating if desired). The spray system 8 may, for example, comprise the spray system with a gantry as disclosed in United States Patent Nos. 6,387,081 and 7,462,242. In this implementation, the air outlet 4 with a single opening 20 may be provided to move as well, for example, vertically along the same or different gantry track.

On one side of the enclosure, preferably opposite the side which supports an entry door 26 enabling the human target to enter the enclosure, an exhaust fan 15 is provided. This exhaust fan 15 operates to exhaust air, spray particles, etc., from inside the enclosure. The exhaust fan 15 draws the air, spray particles, etc., from inside the enclosure through an inlet 14 to outside of the enclosure. The exhaust fan 15 can further be operated to control temperature within the enclosure by controlling the amount of heated air which is retained within the enclosure (for example, by controlling a rate of venting). The exhaust fan 15 can further be used at the end of a spray session to assist with the cleaning of the inside of the enclosure (for example, by removing excess moisture and fumes from cleaning solution and assisting with drying). Alternatively, an active fan need not be activated for exhausting the enclosure, but rather a venting system (perhaps including a filter) could be used.

The system further includes a controller 10 which controls operation of the system. More specifically, the controller 10 controls operation of the heated forced air system 2, the spray system 8 and the exhaust system 15 in a coordinated manner to provide for a more comfortable and efficient spray experience for the human target.

Although FIGURES 1A-1B both show the use of a booth enclosure, it will be understood that this implementation is exemplary only. As an alternative, a fully enclosed environment is not required. In such an alternative embodiment, one or more of the walls could be omitted in a partially enclosed environment. Alternatively, the door could be omitted. The partially enclosed environment in this implementation would still provide for and support the formation of a heated spray environment. Such a configuration may be more acceptable and inviting to customers who suffer from, for example, claustrophobia. In this configuration, the presence of a roof, even with one or more open walls, is preferred so as to retain sufficient heat and support the formation of a heated spray environment. It will further be understood that the spray system and heated forced air system can be provided and mounted within a room, where the walls of the room serve to form the enclosure and define the heated spray environment.

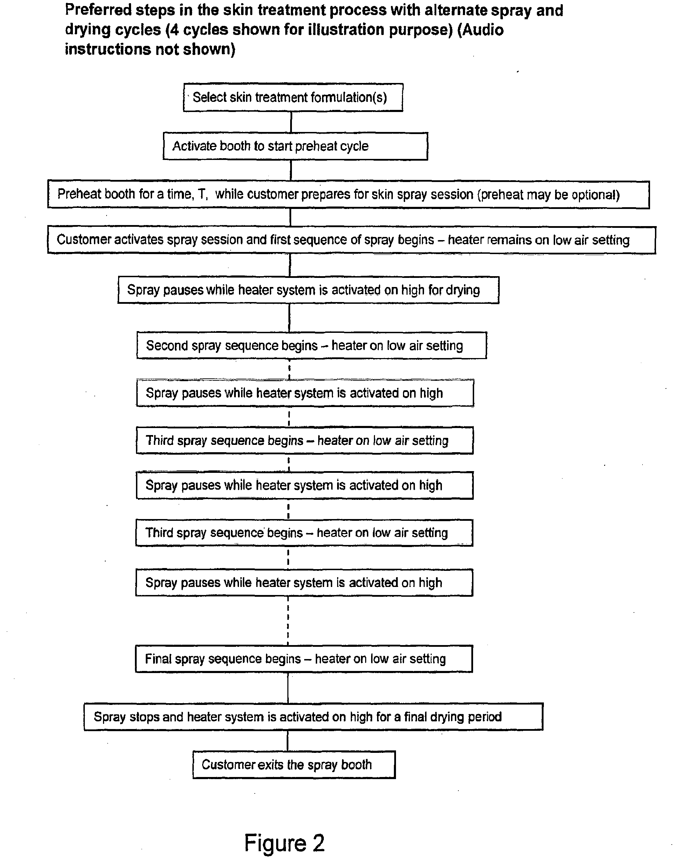

Reference is now made to FIGURE 2 which is a flow direction for an exemplary operation of the spray system. The system is initially activated to start a pre-heat cycle. During this pre-heat cycle, which lasts for a predetermined time period (for example, the expected time taken by the human target to disrobe in preparation for the spray session), the controller 10 activates the heated forced air system 2 to deliver heated air into the enclosure. Thus, the inside of the enclosure will be warmed prior to entry of the human target. It will be recognized that the pre-heat cycle is optional. In this pre-heat cycle, the fan 13 may be activated at a low-speed setting and the heating element may be activated on a high or low setting to control the heat density of the air supplied into the enclosure. The exhaust system 15 may also be activated in concert with the heated forced air system 2 to control setting of the inside enclosure air temperature. An appropriate temperature sensor (not shown) may be included to provide temperature feedback to the controller 10 during the pre-heat cycle.

Before or while the pre-heat cycle is being performed, the customer selects one or more skin treatments to be performed. For example, the customer may choose a moisturizer treatment, a sunless tanning treatment and a skin vitamin or anti-aging treatment. A spray session, as described below and shown in FIGURE 2, is then performed successively with respect to each of the selected skin treatments.

Next, the human target enters the enclosure and initiates a spray session. The controller 10 activates the heated forced air system 2 to deliver heated air into the enclosure and towards the human target and further simultaneously activates the spray system 8 to deliver spray materials towards the human target. Preferably, the fan 13 is activated at a low-speed setting during this step so as to deliver warm air during spraying of the material, but with a force that will not adversely affect the delivery of that spray material. Additionally, the heating element is controlled on its higher heat setting so as to increase the heat density of the air being supplied during spraying. Furthermore, if the heating element was set at the higher level during the pre-heat cycle, the heating element (such as a finned element with sufficient heat storing mass) functions to store heat and make that stored heat more readily available for delivery during a short subsequent period of heated higher air flow. So, the fan can be selectively activated at higher speed, if desired, during or immediately after the spraying operation and because of the stored heat in the heating element an air flow at desired higher temperature can be maintained. It will be understood that in an alternative implementation, the simultaneous delivery of low speed heated air by the heated forced air system 2 is optional. Operation of the spray system 8 includes moving the spray cloud over the human target, for example, by moving the spray nozzle 9 with an oscillating effect and/or vertically traversing the nozzle using the gantry 22.

Following completion of the delivery of spray materials, the controller 10 deactivates the spray system 8 and switches activation of the heated forced air system 2 from low speed to high speed in order to deliver heated air towards the human target for the purpose of drying the skin. If the heated forced air system 2 was previously off during the spray operation, then the controller 10 activates the heated forced air system 2 at high speed in order to deliver heated air towards the human target for the purpose of drying the skin. Conversely, if the heating element was on during spraying, the heat stored by the heating element is made available when the forced air system switches to high speed so as to maintain an acceptable higher temperature level during the drying cycle.

The controller 10 may also monitor the temperature inside the enclosure during the drying step and activate the exhaust system 15, if necessary, to control the temperature at a level comfortable to the human target and reduce the likelihood of perspiration which could interfere with the effective delivery and absorption of spray materials. The exhaust system 15 may also be activated to assist in removing aerosol spray components from within the enclosure during the drying step so as enhance human target comfort during extended multiple step spray sessions.

The foregoing two operations of spray and dry are then iteratively repeated, if necessary, as many times as desired to achieve a desired coating of the human target with the selected spray material. Thus, each iteration includes a spray of material (optionally including a simultaneous low speed heated air application), followed by a high speed heated air application for drying the sprayed material (optionally including a simultaneous exhausting operation).

Each iteration may be accompanied by a different orientation of the human target. Thus, in a first spray/dry sequence, the human target may stand oriented facing the spray nozzle on the first and third standing indicators 17. In a second spray/dry sequence, the human target may stand oriented facing the spray nozzle on the second and fourth standing indicators 17. In a third spray/dry sequence, the human target may stand oriented with his back facing the spray nozzle on the third and first standing indicators 17. In a fourth spray/dry sequence, the human target may stand oriented with his back facing the spray nozzle on the fourth and second standing indicators 17. In this way, substantially complete and even coverage of the human target with spray material is possible. The controller 10 can direct the orientation of the human target by issuing audible instructions to the human target through a speaker 18 located within the enclosure.

Following completion of spraying and drying at each desired orientation of the human target, and the completion of spraying of each selected material, the spray operation terminates allowing the human target to exit the enclosure. Further operations associated with cleaning and maintenance may then be performed under the control of the controller 10. Such operations may include activating the spray system 8 to purge fluid lines, activation of the spray system 8 or separate rinse system (not explicitly shown) to deliver a cleaning solution within the enclosure, activation of the forced air system 2 (with or without heating the air) to dry the inside of the enclosure, and activation of the exhaust fan 15 to exhaust air, spray particles, etc., from inside the enclosure. A sump is preferably included in the floor to capture rinsate, overspray, purge, etc. As an example, the system is controlled when effectuating the drying of the booth to set the heating element at a lower temperature (with higher fan speed) which allows for drying air to be supplied within the enclosure over a longer period of time without risking damaging the heating element and wasting electricity.

It will further be understood that the second operation discussed above, where the controller 10 deactivates the spray system 8 and activates the heated forced air system 2 at high speed in order to deliver heated air towards the human target for the purpose of drying the skin, may be accompanied by activation of exhaust fan 15 to exhaust air, spray particles, etc., from inside the enclosure. This actuation will remove aerosols from the enclosure and improve human target comfort (as many users will hold their breath during the spraying step and will not want to inhale residual aerosol components during the drying step).

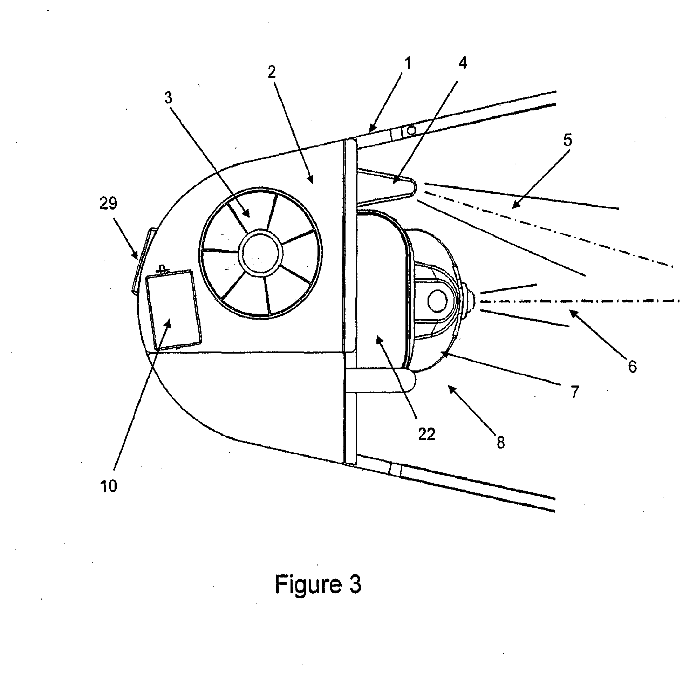

Reference is now made to FIGURE 3 which shows a close-up view of the heated forced air system and spray system within the enclosure of FIGURE 1. FIGURE 3 illustrates an exemplary positional relationship between the air outlet 4 and the spray nozzle on the wall panel, and more specifically shows the orientation of the warm air stream 5 to intersect the spray cloud 6, with the air stream and spray cloud originating from the same general direction. It will be noted that the air outlet is positioned adjacent the spray system.

FIGURE 3 further shows the placement of a touch pad 29 through which a customer interfaces with the system. Through this pad 29, the customer can choose the solution to be sprayed, which portion of body is to sprayed, order of solution spraying, language of the audible instructions. The pad 29 is further used to control maintenance and cleaning operations. The pad further includes indicators (lights, displays, etc.) which can confirm instructions and provide information concerning system operation (faults, scheduled maintenance, solution refill/replacement, etc.).

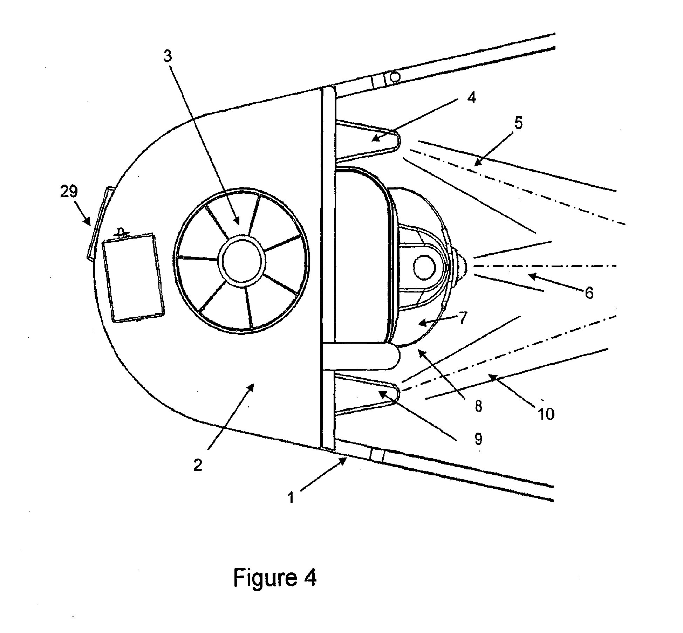

Reference is now made to FIGURE 4 which shows a close-up view of an alternative implementation of the heated forced air system and spray system within the enclosure. In this implementation, the panel wall includes first and second air outlets 4 and 9 located on either side of the nozzle housing 7. The outlet 9 is configured in a manner like the outlet 4, adjacent the spray system but on an opposite side from the outlet 4. The outlets 4 and 9 are supplied heated forced air from the heated forced air system 2. Heated air is forced by the fan of system 2 through the air outlet 4 to form a first warm air stream 5 delivered to the inside of the enclosure. Heated air is also forced by the fan of system 2 through the air outlet 9 to form a second warm air stream 10 delivered to the inside of the enclosure. The air outlets 4 and 9 are configured to direct the warm air stream towards an area 12 (see, FIGURE 1B) where the human target is standing and at about that location the streams intersect with the spray cloud 6. Again, the warm air streams and the spray cloud originate from the same general direction.

Referring now to FIGURES 1A-4, a booth of suitable size is provided with walls 1 for enclosing the human target to be sprayed. A preferred embodiment of the booth includes a door and a roof. A spray system 8 is positioned to spray the human target. This spray system may comprise, for example, a gantry spray system that moves the spray nozzle vertically to pass the spray cloud 6 uniformly over the human target or selected parts of the human target. A fan and heater system 2 is provided to deliver warm (heated) air within the booth. It is preferred that the warm air is directed (by outlets 4 and/or 9) towards the human target and further directed so as to mix with the cloud 6 of spray mist. The fan system 2 preferably draws fresh air in from the outside of the spray enclosure through an air inlet 3. An exhaust system 15 is provided to filter air exiting the booth. In a preferred implementation, the exhaust system 15 is controlled such that its fan selectively operates so as to help stabilize temperature inside the booth (e.g., by actuating to exhaust colder air and retain warmer air in the enclosure, or draw colder air into the enclosure to mix with warmer air). A controller 10 is provided to control operation of the fan and heater system 3 such that its plenum fan and/or heating elements function to pre-warm the booth prior to entry of the human target and initiation of a spray cycle. The controller 10 further controls the fan, heater and spray systems 2 and 8 to iteratively alternate between spraying and drying cycles (with or without differing human target orientations). It is preferable to have the controller 10 provide for a longer drying cycle at the end of a given spraying session. The controller 10 may further implement an automatic booth rinsing system that controls the heater and fan to dry the booth after a rinsing operation.

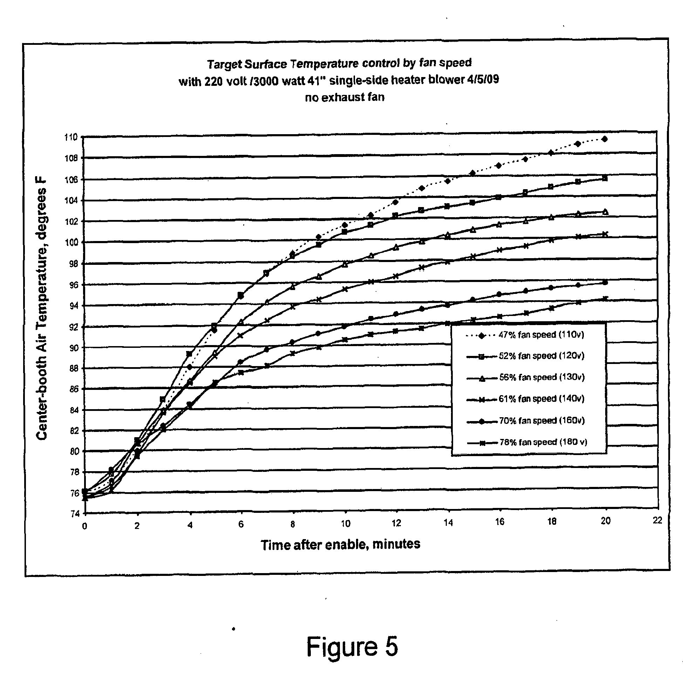

The fan and heater system 3 produces warm dry air within the spray booth enclosure and directs that air towards the human target. One aspect of a preferred implementation orients the supplied warm air stream 5 to mix with the spray cloud 6 during the spray event. The fan and heater system 3 includes a fan actuated to move ambient air past a finned heating element (not specifically illustrated) within a plenum 28 to both dry and heat the air. The plenum is used to equalize air pressure behind each of the slots 20 in the air outlet 4, it being understood that the slots may have different lengths and widths to provide for differing air velocities and air volumes as applied towards the human target. The finned heating element is designed to heat quickly, achieving the desired temperature in the short time it takes for the customer to disrobe and prepare for a spray session (as discussed above with respect to the pre-heat cycle). The fan and heater system 3 is operated by the controller 10 in a manner which integrates heater system operation to the operation of the sprayer mechanism and other components of the booth, such as an exhaust fan system and an automatic rinse and clean system. It is preferred that the fan and heater system 3 have a variable speed so as to enable temperature control and variable airflow effects for the cycles of preheating, spraying and/or drying. FIGURE 5 illustrates a graph of temperature control in relation to fan speed. For example, control of the fan and heater system 3 is made for a lower airflow during spraying and a higher airflow during the drying cycle.

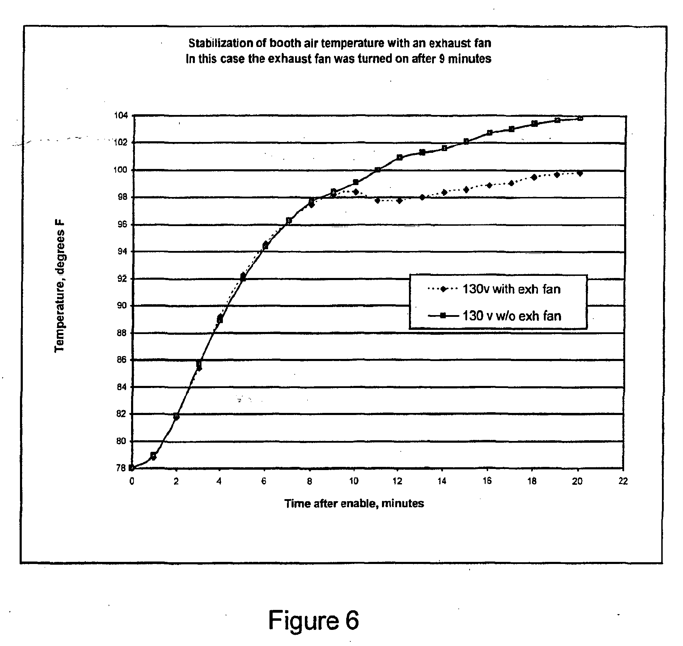

In a preferred implementation, ambient air is pulled into the plenum of the fan and heater system 3 and forced under pressure into the booth. This has the effect of reducing the aerosol content of the air during the spray process. The preferred booth embodiment has an exhaust fan and filter system 15 or more simply uses a filtered vent. The exhaust fan and filter system (or vent) removes spray particles before exhausting to the outside of the booth. An additional fan or vent can be used to improve filtration and stabilize the final temperature of the booth. FIGURE 6 illustrates a graph of final temperature stabilization using an exhaust fan.

The preferred embodiment of the spray booth is a complete enclosure with a door and roof to enable efficient pre-warming of the booth and also efficient warming and containment of spray during the spray cycle. The preferred plenum design has one or more outlets 4 and/or 9 that project the warm air toward the human target. The outlet 4 and/or 9 directs the air causing it to intersect the spray and mix with the spray cloud 6 in transit to the target, thus helping to warm the spray prior to contact with the human target. The preferred outlet 4 and/or 9 design is long and narrow in size, shape and orientation so as to direct the warm air over a substantial portion of the length of the human target. It will be understood, however, that alternatively the outlets 4 and/or 9 may oscillate, vertically travel, or utilize moving louvers to sweep the air over the human target.

The installation of radiant heat lamps in (or near) an automated spray booth is known in the art. However, the use of radiant heaters, such as radiant heat lamps, has been found to be less effective than the forced dry air method as described herein for improving the spray tanning experience. Radiant heat lamps do not heat the ambient air and skin surface as quickly and can cause perspiration before effective temperatures are reached. Furthermore, spray residue can fall on and dry quickly on the lamp's glass surface. Once baked on, this dry material is very difficult to clean and remove. In addition, the glass composition can present a safety hazard from breakage due to impact or rapid cooling if spray or rinse water contacts the surface. In addition, heat lamp methods do not substantially warm the spray mist or improve the ambient air quality of the spray booth compared to the forced-air methods.

Improved results with the systems and methods described herein have been observed during trials with DHA (dihydroxyacetone) based sunless tanning compounds. The improved results include: increased tan color by allowing higher quantities of sprayed active ingredient to be deposited due to a layering process where the spray is applied, then the skin is re-dried quickly by the warm air flow before another spray pass; promotion of deeper activity of DHA by drying the top layer of skin completely and possibly by drying inner layers of the stratum corneum skin layer, resulting in longer lasting tan color; opening of skin surface pores to allow for better penetration of tanning compound and skin care ingredients; drying the skin of perspiration or other moisture, including the water based spray itself, that may cause an uneven tanning effect and prevent penetration into skin layers; preventing dripping or streaking of the sprayed material during the tanning process which can cause an uneven tanning result; eliminating the step of drying the skin off with a towel which causes partial removal and disturbance of the evenly deposited layer from the spray application; and reducing presence of lingering aerosols in the booth environment by the introduction of outside air through the forced air system.

It should be emphasized that the terms "comprise", "comprises", and "comprising", when used herein, are taken to specify the presence of stated features, integers, steps, or components, but do not preclude the presence or addition of one or more other features, integers, steps, components or groups thereof.

In the foregoing Detailed Description, it can be seen that various features may be grouped together in a single embodiment for the purpose of streamlining the disclosure. This method of disclosure is not to be interpreted as reflecting an intention that the claimed embodiments of the invention require more features than are expressly recited in each claim. Rather, as the following claims reflect, inventive subject matter lies in less than all features of a single disclosed embodiment. Thus the following claims are hereby incorporated into the Detailed Description, with each claim standing on its own as a separate embodiment. The scope of the invention is defined by the following claims and the equivalents thereof.

## Claims

1. A method for application of a non therapeutical skin treatment spray solution to a human target, comprising:
a) spraying the non therapeutical skin treatment spray solution from a nozzle (9) within a spray system (8);
b) terminating nozzle spraying;
c) blowing air from a forced air system (2) through an air outlet (4) that is positioned adjacent to the spray system to dry the sprayed skin treatment spray solution;
performing steps a)-c) under the control of an automated control system (10) coupled to the spray system and the forced air system; **characterized in that** a) spraying further comprises simultaneously activating the forced air system to blow air while spraying the skin treatment spray solution.

2. The method of claim 1 further comprising d) repeating steps a)-c) a desired number of times under the control of the automated control system.

3. The method of claim 1 further comprising performing steps a)-d) within an enclosure.

4. The method of claim 3, wherein the blown air is drawn from outside of the enclosure and delivered into the enclosure; or
further comprising, prior to step a), activating the forced air system to blow heated air into the enclosure; or
further comprising, after step d), activating an exhaust system (15) to forcibly exhaust air and spray solution from within the enclosure.

5. The method of claim 1 wherein c) blowing air further comprises activating a heater (2,3) within the forced air system to heat the blown air.

6. The method of claim 1 wherein simultaneously blowing air further comprises activating a heater (2,3) within the forced air system to heat the simultaneously blown air.

7. The method of claim 6 wherein a) spraying further comprises directing the simultaneously blown air to mix with a spray cloud (6) formed by the sprayed skin treatment spray solution; or
further comprising activating an exhaust system (15) to forcibly exhaust air from within the enclosure to control a temperature within the enclosure.

8. The method of claim 1 wherein a) spraying generates a spray cloud (6) of skin treatment spray solution and wherein a) spraying further comprises moving the spray cloud.

9. The method of claim 8 wherein moving comprises horizontally oscillating the nozzle within a spray system (8), or wherein moving comprises vertically traversing the spray system.

10. The method of claim 1 wherein blowing air from the forced air system through the air outlet further comprised providing a vertical temperature gradient with respect to the blown air.

11. The method of claim 10 wherein the temperature gradient provides for relatively cooler air at higher vertical positions and relatively warmer air at lower vertical positions.

12. Apparatus, comprising:
a spray system (8) including a spray nozzle (9) for spraying a skin treatment spray solution;
a forced air system (2) for blowing air;
an air outlet (4) positioned adjacent to the spray system and coupled to the forced air system;
an automated control system (10) which is configured to operate to:
a) cause the spray system to spray the skin treatment spray solution through the nozzle;
b) terminate operation of the spray system; and
c) activate the forced air system to blow air through the air outlet to dry the sprayed skin treatment spray solution; **characterized in that**
the automated control system is further configured to simultaneously activate the spray system and the forced air system to blow air while spraying with the skin treatment spray solution.

13. The apparatus of claim 12 wherein the automated control system is further configured to d) repeat operations a)-c) a desired number of times.

14. The apparatus of claim 12 further comprising an enclosure for enclosing the spray system and the forced air system.

15. The apparatus of claim 14 wherein the blown air is drawn from outside of the enclosure and delivered into the enclosure; or
wherein the automated control system is further configured to activate the forced air system to blow heated air into the enclosure prior to operation a); or
further comprising an exhaust system (15) operating under the control of the automated control system to exhaust air and spray solution from within the enclosure.

16. The apparatus of claim 12 wherein the forced air system is activated to blow heated air.

17. The apparatus of claim 12 wherein the forced air system is activated to blow heated air.

18. The apparatus of claim 17 wherein the forced air system directs the simultaneously blown air to mix with a spray cloud (6) formed by the sprayed skin treatment spray solution, or
further comprising an exhaust system (15) operating under the control of the automated control system to exhaust air from within the enclosure to control a temperature within the enclosure.

19. The apparatus of claim 12 wherein the spray system generates a spray cloud (6) of skin treatment spray solution, further comprising means for moving the spray cloud.

20. The apparatus of claim 19 wherein the means for moving horizontally oscillates the nozzle of the spray system, or wherein the means for moving vertically traverses the spray system.

21. The apparatus of claim 12 wherein the air outlet positioned adjacent to the spray system comprises at least one vertically extending slot (4,20).

22. The apparatus of claim 12 wherein the spray nozzle is an electrostatic nozzle.

23. The apparatus of claim 12 wherein the air outlet produces an air stream (5) and the spray nozzle produces a spray cloud (6), the air outlet configured to direct the air stream to intersect with the spray cloud.

24. The apparatus of claim 12 wherein the air outlet supports provision of a vertical temperature gradient with respect to the blown air.

25. The apparatus of claim 24 wherein the temperature gradient provides for relatively cooler air at higher vertical positions and relatively warmer air at lower vertical positions.

## Patentansprüche

1. Ein Verfahren zum Auftragen einer nicht therapeutischen Hautbehandlungssprühlösung auf eine Zielperson, das Folgendes beinhaltet:
a) Sprühen der nicht therapeutischen Hautbehandlungssprühlösung aus einer Düse (9) innerhalb eines Sprühsystems (8);
b) Beenden des Düsensprühens;
c) Blasen von Luft aus einem Gebläseluftsystem (2) durch einen Luftauslass (4), der neben dem Sprühsystem positioniert ist, um die gesprühte Hautbehandlungssprühlösung zu trocknen;
Durchführen der Schritte a)-c) unter der Steuerung eines automatisierten Steuerungssystems (10), das an das Sprühsystem und das Gebläseluftsystem gekoppelt ist; **dadurch gekennzeichnet, dass**
a) das Sprühen ferner das gleichzeitige Aktivieren des Gebläseluftsystems beinhaltet, um Luft zu blasen, während die Hautbehandlungssprühlösung gesprüht wird.

2. Verfahren gemäß Anspruch 1, das ferner d) das Wiederholen der Schritte a)-c) für eine gewünschte Anzahl an Malen unter der Steuerung des automatisierten Steuerungssystems beinhaltet.

3. Verfahren gemäß Anspruch 1, das ferner das Durchführen der Schritte a)-d) innerhalb einer Kabine beinhaltet.

4. Verfahren gemäß Anspruch 3, wobei die geblasene Luft von außerhalb der Kabine gezogen und in die Kabine abgegeben wird; oder
das ferner vor Schritt a) das Aktivieren des Gebläseluftsystems zum Blasen von erwärmter Luft in die Kabine beinhaltet; oder
das ferner nach Schritt d) das Aktivieren eines Absaugsystems (15) zum kraftvollen Absaugen von Luft und Sprühlösung aus dem Inneren der Kabine beinhaltet.

5. Verfahren gemäß Anspruch 1, wobei c) das Blasen von Luft ferner das Aktivieren einer Heizung (2, 3) innerhalb des Gebläseluftsystems zum Erwärmen der geblasenen Luft beinhaltet.

6. Verfahren gemäß Anspruch 1, wobei das gleichzeitige Blasen von Luft ferner das Aktivieren einer Heizung (2, 3) innerhalb des Gebläseluftsystems zum Erwärmen der gleichzeitig geblasenen Luft beinhaltet.

7. Verfahren gemäß Anspruch 6, wobei a) das Sprühen ferner das Leiten der gleichzeitig geblasenen Luft zum Mischen mit einer Sprühnebelwolke (6), die von der gesprühten Hautbehandlungssprühlösung gebildet wird, beinhaltet; oder
das ferner das Aktivieren eines Absaugsystems (15) zum kraftvollen Absaugen von Luft aus dem Inneren der Kabine zum Steuern einer Temperatur innerhalb der Kabine beinhaltet.

8. Verfahren gemäß Anspruch 1, wobei a) das Sprühen eine Sprühnebelwolke (6) aus Hautbehandlungssprühlösung erzeugt und wobei a) das Sprühen ferner das Bewegen der Sprühnebelwolke beinhaltet.

9. Verfahren gemäß Anspruch 8, wobei das Bewegen das horizontale Hin- und Herbewegen der Düse innerhalb eines Sprühsystems (8) beinhaltet oder wobei das Bewegen das vertikale Verschieben des Sprühsystems beinhaltet.

10. Verfahren gemäß Anspruch 1, wobei das Blasen von Luft aus dem Gebläseluftsystem durch den Luftauslass ferner das Bereitstellen eines vertikalen Temperaturgradienten in Bezug auf die geblasene Luft beinhaltet.

11. Verfahren gemäß Anspruch 10, wobei der Temperaturgradient für relativ kühlere Luft in höheren vertikalen Positionen und relativ wärmere Luft in niedrigeren vertikalen Positionen sorgt.

12. Eine Vorrichtung, die Folgendes beinhaltet:
ein Sprühsystem (8), das eine Sprühdüse (9) zum Sprühen einer Hautbehandlungssprühlösung umfasst;
ein Gebläseluftsystem (2) zum Blasen von Luft;
einen Luftauslass (4), der neben dem Sprühsystem positioniert und an das Gebläseluftsystem gekoppelt ist;
ein automatisiertes Steuerungssystem (10), das konfiguriert ist, um für Folgendes in Betrieb zu sein:
a) Bewirken, dass das Sprühsystem die Hautbehandlungssprühlösung durch die Düse sprüht;
b) Beenden des Betriebs des Sprühsystems; und
c) Aktivieren des Gebläseluftsystems, um Luft durch den Luftauslass zu blasen, um die gesprühte Hautbehandlungssprühlösung zu trocknen; **dadurch gekennzeichnet, dass**
das automatisierte Steuerungssystem ferner konfiguriert ist, um das Sprühsystem und
das Gebläseluftsystem gleichzeitig zu aktiveren, um Luft zu blasen, während die Hautbehandlungssprühlösung gesprüht wird.

13. Vorrichtung gemäß Anspruch 12, wobei das automatisierte Steuerungssystem ferner konfiguriert ist, um d) Vorgänge a)-c) eine gewünschte Anzahl an Malen zu wiederholen.

14. Vorrichtung gemäß Anspruch 12, die ferner eine Kabine zum Einschließen des Sprühsystems und des Gebläseluftsystems beinhaltet.

15. Vorrichtung gemäß Anspruch 14, wobei die geblasene Luft von außerhalb der Kabine gezogen und in die Kabine abgegeben wird; oder
wobei das automatisierte Steuerungssystem ferner konfiguriert ist, um vor dem Vorgang a) das Gebläseluftsystem zum Blasen von erwärmter Luft in die Kabine zu aktivieren; oder
die ferner ein Absaugsystem (15) beinhaltet, das unter der Steuerung des automatisierten Steuerungssystems zum Absaugen von Luft und Sprühlösung aus dem Inneren der Kabine in Betrieb ist.

16. Vorrichtung gemäß Anspruch 12, wobei das Gebläseluftsystem aktiviert wird, um erwärmte Luft zu blasen.

17. Vorrichtung gemäß Anspruch 12, wobei das Gebläseluftsystem aktiviert wird, um erwärmte Luft zu blasen.

18. Vorrichtung gemäß Anspruch 17, wobei das Gebläseluftsystem die gleichzeitig geblasene Luft zum Mischen mit einer Sprühnebelwolke (6), die von der gesprühten Hautbehandlungssprühlösung gebildet wird, leitet, oder
die ferner ein Absaugsystem (15) beinhaltet, das unter der Steuerung des automatisierten Steuerungssystems zum Absaugen von Luft aus dem Inneren der Kabine in Betrieb ist, um eine Temperatur innerhalb der Kabine zu steuern.

19. Vorrichtung gemäß Anspruch 12, wobei das Sprühsystem eine Sprühnebelwolke (6) aus Hautbehandlungssprühlösung erzeugt, ferner beinhaltend ein Mittel zum Bewegen der Sprühnebelwolke.

20. Vorrichtung gemäß Anspruch 19, wobei das Mittel zum Bewegen die Düse des Sprühsystems horizontal hin- und herbewegt oder wobei das Mittel zum Bewegen das Sprühsystem vertikal verschiebt.

21. Vorrichtung gemäß Anspruch 12, wobei der neben dem Sprühsystem positionierte Luftauslass mindestens einen sich vertikal erstreckenden Schlitz (4, 20) beinhaltet.

22. Vorrichtung gemäß Anspruch 12, wobei die Sprühdüse eine elektrostatische Düse ist.

23. Vorrichtung gemäß Anspruch 12, wobei der Luftauslass einen Luftstrom (5) produziert und die Sprühdüse eine Sprühnebelwolke (6) produziert, wobei der Luftauslass konfiguriert ist, um den Luftstrom so zu leiten, dass er die Sprühnebelwolke kreuzt.

24. Vorrichtung gemäß Anspruch 12, wobei der Luftauslass die Bereitstellung eines vertikalen Temperaturgradienten in Bezug auf die geblasene Luft unterstützt.

25. Vorrichtung gemäß Anspruch 24, wobei der Temperaturgradient für relativ kühlere Luft in höheren vertikalen Positionen und relativ wärmere Luft in niedrigeren vertikalen Positionen sorgt.

## Revendications

1. Une méthode pour appliquer une solution de pulvérisation pour traitement cutané non thérapeutique sur une cible humaine, comprenant le fait de :
a) pulvériser la solution de pulvérisation pour traitement cutané non thérapeutique depuis une buse (9) au sein d'un système de pulvérisation (8) ;
b) terminer la pulvérisation par buse ;
c) souffler de l'air depuis un système à air forcé (2) par une sortie d'air (4) qui est positionnée adjacente au système de pulvérisation pour sécher la solution de pulvérisation pour traitement cutané pulvérisée ;
réaliser les étapes a) à c) sous le contrôle d'un système de contrôle automatisé (10) couplé au système de pulvérisation et au système à air forcé ; **caractérisée en ce que** la pulvérisation a) comprend en outre le fait d'activer de façon simultanée le système à air forcé pour souffler de l'air tout en pulvérisant la solution de pulvérisation pour traitement cutané.

2. La méthode de la revendication 1 comprenant en outre d) le fait de répéter les étapes a) à c) un nombre souhaité de fois sous le contrôle du système de contrôle automatisé.

3. La méthode de la revendication 1 comprenant en outre le fait de réaliser les étapes a) à d) dans une enceinte.

4. La méthode de la revendication 3, dans laquelle l'air soufflé est extrait de l'extérieur de l'enceinte et amené dans l'enceinte ; ou
comprenant en outre, avant l'étape a), le fait d'activer le système à air forcé pour souffler de l'air chauffé dans l'enceinte ; ou
comprenant en outre, après l'étape d), le fait d'activer un système d'évacuation (15) pour évacuer de façon forcée air et solution de pulvérisation du dedans de l'enceinte.

5. La méthode de la revendication 1 dans laquelle le soufflage d'air c) comprend en outre le fait d'activer un organe de chauffage (2, 3) au sein du système à air forcé pour chauffer l'air soufflé.

6. La méthode de la revendication 1 dans laquelle le soufflage d'air de façon simultanée comprend en outre le fait d'activer un organe de chauffage (2, 3) dans le système à air forcé pour chauffer l'air soufflé de façon simultanée.

7. La méthode de la revendication 6 dans laquelle la pulvérisation a) comprend en outre le fait de diriger l'air soufflé de façon simultanée de façon à le mélanger à un nuage de pulvérisation (6) formé par la solution de pulvérisation pour traitement cutané pulvérisée ; ou
comprenant en outre le fait d'activer un système d'évacuation (15) pour évacuer de façon forcée de l'air du dedans de l'enceinte de façon à contrôler une température dans l'enceinte.

8. La méthode de la revendication 1 dans laquelle la pulvérisation a) génère un nuage de pulvérisation (6) de solution de pulvérisation pour traitement cutané et dans laquelle la pulvérisation a) comprend en outre le fait de déplacer le nuage de pulvérisation.

9. La méthode de la revendication 8 dans laquelle le déplacement comprend le fait de faire osciller horizontalement la buse au sein d'un système de pulvérisation (8), ou dans laquelle le déplacement comprend le fait de traverser verticalement le système de pulvérisation.

10. La méthode de la revendication 1 dans laquelle le soufflage d'air depuis le système à air forcé par la sortie d'air comprend en outre le fait de fournir un gradient de température vertical par rapport à l'air soufflé.

11. La méthode de la revendication 10 dans laquelle le gradient de température procure un air relativement plus frais à des positions verticales plus hautes et un air relativement plus chaud à des positions verticales plus basses.

12. Appareil, comprenant :
un système de pulvérisation (8) comportant une buse de pulvérisation (9) destinée à pulvériser une solution de pulvérisation pour traitement cutané ;
un système à air forcé (2) destiné à souffler de l'air ;
une sortie d'air (4) positionnée adjacente au système de pulvérisation et couplée au système à air forcé ;
un système de contrôle automatisé (10) qui est configuré de façon à opérer pour :
a) amener le système de pulvérisation à pulvériser la solution de pulvérisation pour traitement cutané par la buse ;
b) terminer l'opération du système de pulvérisation ; et
c) activer le système à air forcé pour souffler de l'air par la sortie d'air afin de sécher la solution de pulvérisation pour traitement cutané pulvérisée ; **caractérisé en ce que** le système de contrôle automatisé est configuré en outre de façon à activer simultanément le système de pulvérisation et le système à air forcé pour souffler de l'air tout en pulvérisant avec la solution de pulvérisation pour traitement cutané.

13. L'appareil de la revendication 12 dans lequel le système de contrôle automatisé est configuré en outre de façon à d) répéter les opérations a) à c) un nombre souhaité de fois.

14. L'appareil de la revendication 12 comprenant en outre une enceinte destinée à enfermer le système de pulvérisation et le système à air forcé.

15. L'appareil de la revendication 14 dans lequel l'air soufflé est extrait de l'extérieur de l'enceinte et amené dans l'enceinte ; ou
dans lequel le système de contrôle automatisé est configuré en outre de façon à activer le système à air forcé pour qu'il souffle de l'air chauffé dans l'enceinte avant l'opération a) ; ou
comprenant en outre un système d'évacuation (15) opérant sous le contrôle du système de contrôle automatisé pour évacuer air et solution de pulvérisation du dedans de l'enceinte.

16. L'appareil de la revendication 12 dans lequel le système à air forcé est activé pour souffler de l'air chauffé.

17. L'appareil de la revendication 12 dans lequel le système à air forcé est activé pour souffler de l'air chauffé.

18. L'appareil de la revendication 17 dans lequel le système à air forcé dirige l'air soufflé de façon simultanée pour le mélanger à un nuage de pulvérisation (6) formé par la solution de pulvérisation pour traitement cutané pulvérisée, ou
comprenant en outre un système d'évacuation (15) opérant sous le contrôle du système de contrôle automatisé pour évacuer de l'air du dedans de l'enceinte de façon à contrôler une température dans l'enceinte.

19. L'appareil de la revendication 12 dans lequel le système de pulvérisation génère un nuage de pulvérisation (6) de solution de pulvérisation pour traitement cutané, comprenant en outre un moyen pour déplacer le nuage de pulvérisation.

20. L'appareil de la revendication 19 dans lequel le moyen de déplacement horizontal fait osciller la buse du système de pulvérisation, ou dans lequel le moyen de déplacement vertical traverse le système de pulvérisation.

21. L'appareil de la revendication 12 dans lequel la sortie d'air positionnée adjacente au système de pulvérisation comprend au moins une fente s'étendant verticalement (4, 20).

22. L'appareil de la revendication 12 dans lequel la buse de pulvérisation est une buse électrostatique.

23. L'appareil de la revendication 12 dans lequel la sortie d'air produit un flux d'air (5) et la buse de pulvérisation produit un nuage de pulvérisation (6), la sortie d'air étant configurée de façon à diriger le flux d'air pour qu'il coupe le nuage de pulvérisation.

24. L'appareil de la revendication 12 dans lequel la sortie d'air soutient l'apport d'un gradient de température vertical par rapport à l'air soufflé.

25. L'appareil de la revendication 24 dans lequel le gradient de température procure un air relativement plus frais à des positions verticales plus hautes et un air relativement plus chaud à des positions verticales plus basses.
